# EUROPEAN PATENT APPLICATION

(11) **EP 3 069 717 A1**
(43) Date of publication of application: **21.09.2016**
(21) Application number: 16161025.8
(22) Date of filing: 26.04.2010
(51) Int. Cl.: A61K 31/137, A61K 31/215, A61P 25/04

(54) **USE OF 1-PHENYL-3-DIMETHYLAMINOPROPANE COMPOUNDS FOR TREATING RHEUMATOID PAIN**

(30) Priority: 30.04.2009 EP 09005980
(62) Divisional of application: 10721109.6
(71) Applicant: Grünenthal GmbH, 52078 Aachen (DE)
(72) Inventor: JAHNEL, Ulrich, 42855 Remscheid (DE); SCHIENE, Klaus, 41363 Jüchen (DE)
(74) Representative: Kutzenberger Wolff & Partner

(57) **Abstract**

The invention relates to the use of 1-phenyl-3-dimethylaminopropane compounds, such as tapentadol, for the production of medicaments for treating rheumatoid, preferably rheumatoid arthritic, very preferably chronic rheumatoid arthritic pain.

## Description

The present invention relates to the use of 1-phenyl-3-dimethylaminopropane compounds for the production of medicaments for treating rheumatoid, preferably rheumatoid arthritic, very preferably chronic rheumatoid arthritic pain.

Rheumatoid arthritis is a chronic inflammatory disorder, associated with chronic arthritic pain in contrast to acute arthritis disorders like gouty arthritis or septic arthritis, which are associated with acute inflammatory pain. Therefore rheumatoid pain, rheumatoid arthritic pain and rheumatoid chronic arthritic pain are clearly separated from acute inflammatory pain (Wilson et al., 2006).

The object of the present invention was accordingly to provide compounds that are effective in treating rheumatoid, preferably rheumatoid arthritic, very preferably chronic rheumatoid arthritic pain.
This is complicated by the fact that a large proportion of the substances effective in treating nociceptive pain - such as acute pain - are lower effective at all, in treating rheumatoid pain.

It has now surprisingly been found that the compounds disclosed herein below are highly effective in treating rheumatoid pain, and surprisingly particularly effective in treating rheumatoid arthritic and very preferred in treating rheumatoid arthritic chronic pain.

Accordingly, the present invention provides for the use of a 1-phenyl-3-dimethylaminopropane compound according to the general formula I 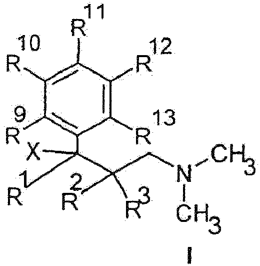
wherein X is selected from OH, F, Cl, OC(O)CH₃ or H, preferably OH, F, OC(O)CH₃ or H,
   and/or
R¹ is selected from C₁₋₄-alkyl, saturated and unsubstituted, branched or unbranched; preferably CH₃, C₂H₅, C₄H₉ or t-butyl, in particular CH₃ or C₂H₅,
   and/or
R² and R³ independently of one another are selected from H, C₁₋₄-alkyl, saturated and unsubstituted, branched or unbranched; preferably H, CH₃, C₂H₅, i-propyl or t-butyl, in particular H or CH₃, preferably R³ = H,
   and/or
R⁹ to R¹³, in which three or four of the radicals R⁹ to R¹³ must correspond to H, are independently of one another selected from H, Cl, F, OH, CF₂H, CF₃ or C₁₋₄-alkyl, saturated and unsubstituted, branched or unbranched; OR¹⁴ or SR¹⁴, where R¹⁴ is selected from C₁₋₃-alkyl, saturated and unsubstituted, branched or unbranched;
preferably H, Cl, F, OH, CF₂H, CF₃, OCH₃ or SCH₃
or R¹² and R¹¹ form a 3,4-OCH=CH ring,
in particular
if R⁹, R¹¹ and R¹³ correspond to H, one of R¹⁰ and R¹² also corresponds to H, while the other is selected from:
   Cl, F, OH, CF₂H, CF₃, OR¹⁴ or SR¹⁴, preferably OH, CF₂H, OCH₃ or SCH₃,
      or
   if R⁹ and R¹³ correspond to H and R¹¹ corresponds to OH, OCH₃, Cl or F, preferably to Cl, then one of R¹⁰ and R¹² also corresponds to H, while the other corresponds to OH, OCH₃, Cl or F, preferably Cl,
      or
   if R⁹, R¹⁰, R¹² and R¹³ correspond to H, R¹¹ is selected from CF₃, CF₂H, Cl or F, preferably F,
      or
   if R¹⁰, R¹¹ and R¹² correspond to H, one of R⁹ and R¹³ also corresponds to H, while the other is selected from OH, OC₂H₅ or OC₃H₇,
   optionally in the form of their racemates, their pure stereoisomers, in particular enantiomers or diastereomers, or in the form of mixtures of the stereoisomers, in particular of the enantiomers or diastereomers, in an arbitrary mixture ratio; in the prepared form or in the form of their acids or their bases or in the form of their salts, in particular the physiologically compatible salts, or in the form of their solvates, in particular the hydrates;
   for the production of a medicament for treating rheumatoid, preferably rheumatoid arthritic, very preferably chronic rheumatoid arthritic pain.

Surprisingly it has been found that the aforementioned substances are extremely effective in the *in vivo* model for chronic rheumatoid arthritic pain by Wilson et al., Pain 2006.

In the context of the present invention alkyl and cycloalkyl radicals are understood to denote saturated and unsaturated (but not aromatic), branched, unbranched and cyclic hydrocarbons, which may be unsubstituted or monosubstituted or polysubstituted. In this connection C₁₋₂-alkyl denotes C1- or C2- alkyl, C₁₋₃-alkyl denotes C1-, C2-or C3-alkyl, C₁₋₄-alkyl denotes C1-, C2-, C3- or C4-alkyl, C₁₋₅-alkyl denotes C1-, C2-, C3-, C4- or C5-alkyl, C₁₋₆-alkyl denotes C1-, C2-, C3-, C4-, C5- or C6-alkyl, C₁₋₇-alkyl denotes C1-, C2-, C3-, C4-, C5-, C6- or C7-alkyl, C₁₋₈-alkyl denotes C1-, C2-, C3-, C4-, C5-, C6-, C7- or C8-alkyl, C₁₋₁₀-alkyl denotes C1-, C2-, C3-, C4-, C5-, C6-, C7-, CS,- C9-or C10-alkyl and C₁₋₁₈-alkyl denotes C1-, C2-, C3-, C4-, C5-, C6-, C7-, C8-, C9-, C10-, C11-, C12-, C13-, C14-, C15-, C16-, C17- or C18-alkyl. In addition C₃₋₄-cycloalkyl denotes C3- or C4-cycloalkyl, C₃₋₅-cycloalkyl denotes C3-, C4 or C5-cycloalkyl, C₃₋₆-cycloalkyl denotes C3-, C4-, C5- or C6-cycloalkyl, C₃₋₇ -cycloalkyl denotes C3-, C4-, C5-, C6- or C7-cycloalkyl, C₃₋₈-cycloalkyl denotes C3-, C4-, C5-, C6-, C7- or C8-cycloalkyl, C₄₋₅-cycloalkyl denotes C4- or C5-cycloalkyl, C₄₋₆-cycloalkyl denotes C4-, C5- or C6-cycloalkyl, C₄₋₇cycloalkyl denotes C4-, C5-, C6- or C7-cycloalkyl, C₅₋₆-cycloalkyl denotes C5- or C6-cycloalkyl and C₅₋₇-cycloalkyl denotes C5-, C6- or C7 -cycloalkyl. With regard to cycloalkyl the term also includes saturated cycloalkyls in which one or two carbon atoms are replaced by a heteroatom S, N or O. The term cycloalkyl however in addition also includes in particular monounsaturated or polyunsaturated, preferably monounsaturated, cycloalkyls without a heteroatom in the ring, provided that the cycloalkyl does not form an aromatic system. The alky and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1, 1-dimethylethyl, pentyl, 1,1-dimethylpropyl, 1,2-dimethylpropyl, 2,2-dimethylpropyl, hexyl, 1-methylpentyl, cyclopropyl, 2-methylcyclopropyl, cyclopropylmethyl, cyclobutyl, cyclopentyl, cyclopentylmethyl, cyclohexyl, cycloheptyl, cyclooctyl, but also adamantyl, CHF₂, CF₃ or CH₂OH as well as pyrazolinone, oxopyrazolinone, [1,4] dioxane or dixolane.

At the same time, in connection with alkyl and cycloalkyl - unless expressly defined otherwise - the term "substituted" within the meaning of the present invention denotes the substitution of at least one (optionally also several) hydrogen atom(s) by F, Cl, Br, I, NH₂, SH or OH, in which "polysubstituted" and "substituted" in the case of polysubstitution is understood to mean that the substitution occurs multiply with the same or different substituents on different as well as on the same atoms, for example triple substitution on the same C atom as in the case of CF₃, or at different sites, as in the case of -CH(OH)-CH=CH-CHCl₂. Particularly preferred substituents in this connection are F, Cl and OH. With regard to cycloalkyl the hydrogen atom may also be replaced by OC₁₋₃-alkyl or C₁₋₃-alkyl (in each case monosubstituted or polysubstituted, or unsubstituted) in particular by methyl, ethyl, n-propyl, i-propyl, CF₃, methoxy or ethoxy.

The term (CH₂)₃₋₆ is understood to denote -CH₂-CH₂-CH₂-, -CH₂-CH₂-CH₂-CH₂-, -CH₂-CH₂-CH₂-CH₂-CH₂- and -CH₂-CH₂-CH₂-CH₂-CH₂-CH₂-, the term (CH₂)₁₋₄ is understood to denote -CH₂, -CH₂-CH₂-, -CH₂-CH₂-CH₂- and -CH₂-CH₂-CH₂-CH₂-, and similarly the term (CH₂)₄-₅ is understood to denote -CH₂-CH₂-CH₂-CH₂-and -CH₂-CH₂-CH₂-CH₂-CH₂-, etc.

An aryl radical is understood to denote ring systems with at least one aromatic ring, but without heteroatoms in even only one of the rings. Examples are phenyl, naphthyl, fluoranthenyl, fluorenyl, tetralinyl or indanyl, in particular 9H-fluorenyl or anthracenyl radicals, which may be unsubstituted or monosubstituted or polysubstituted.

A heteroaryl radical is understood to denote heterocyclic ring systems with at least one unsaturated ring, which may contain one or more heteroatoms from the group nitrogen, oxygen and/or sulphur and may also be monosubstituted or polysubstituted. Examples of heteroaryl compounds that may be mentioned include furan, benzofuran, thiophene, benzothiophene, pyrrole, pyridine, pyrimidine, pyrazine, quinoline, isoquinoline, phthalazine, benzo[1,2,5]thiadiazole, benzothiazole, indole, benzotriazole, benzodioxolane, benzodioxane, carbazole, indole and quinazoline.

The term salt is understood to denote any form of the active constituent according to the invention in which this adopts an ionic form or is charged, and is coupled to a counter ion (a cation or anion) or is present in solution. The term is also understood to include complexes of the active constituent with other molecules and ions, in particular complexes that are complexed via ionic interactions. In particular the term is understood to denote (and this is also a preferred embodiment of the invention) physiologically compatible salts, in particular physiologically compatible salts with cations or bases and physiologically compatible salts with anions or acids or also a salt formed with a physiologically compatible acid or a physiologically compatible cation.

The term physiologically compatible is understood to mean that the substance, in particular the salt as such, is compatible when used in humans or mammals, and therefore for example does not act in a non-physiological manner (e.g. is not toxic).

The term physiologically compatible salt with anions or acids is understood within the meaning of the present invention to denote salts of at least one of the compounds according to the invention - generally protonated, for example on the nitrogen atom - as cation with at least one anion, which are physiologically compatible, especially when used in humans and/or mammals. In particular the term is understood within the meaning of the present invention to denote the salt formed with a physiologically compatible acid, namely salts of the respective active constituent with inorganic or organic acids, which are physiologically compatible, especially when used in humans and/or mammals. Examples of physiologically compatible salts of specific acids are salts of the following: hydrochloric acid, hydrobromic acid, sulphuric acid, methanesulfonic acid, formic acid, acetic acid, oxalic acid, succinic acid, malic acid, tartaric acid, mandelic acid, fumaric acid, lactic acid, citric acid, glutamic acid, 1,1-dioxo-1,2-dihydro 1λ⁶-benzo[3]isothiazol-3-one (saccharinic acid), monomethylsebacic acid, 5-oxo-proline, hexane-1-sulfonic acid, nicotinic acid, 2-, 3- or 4-aminobenzoic acid, 2,4,6-trimethylbenzoic acid, α-lipoic acid, acetylglycine, acetylsalicylic acid, hippuric acid and/or aspartic acid. The hydrochloride salt is particularly preferred.

The term salt formed with a physiologically compatible acid is understood within the meaning of the present invention to denote salts of the respective active constituent with inorganic or organic acids, which are physiologically compatible, especially when used in humans and/or mammals. The hydrochloride is particularly preferred. Examples of physiologically compatible acids include the following: hydrochloric acid, hydrobromic acid, sulphuric acid, methanesulfonic acid, formic acid, acetic acid, oxalic acid, succinic acid, tartaric acid, mandelic acid, fumaric acid, lactic acid, citric acid, glutamic acid, 1,1-dioxo-1,2-dihydrolλ⁶-benzo[3]isothiazol-3-one (saccharinic acid), monomethylsebacic acid, 5-oxo-proline, hexane-1-sulfonic acid, nicotinic acid, 2-, 3- or 4-aminobenzoic acid, 2,4,6-trimethylbenzoic acid, α-lipoic acid, acetylglycine, acetylsalicylic acid, hippuric acid and/or aspartic acid.

The term physiologically compatible salts with cations or bases is understood within the meaning of the present invention to denote salts of at least one of the compounds according to the invention - generally a (deprotonated) acid - as anion with at least one, preferably inorganic, cation, which are physiologically compatible, especially when used in humans and/or mammals. Particularly preferred are the salts of the alkali and alkaline earth metals, but also salts with NH₄⁺, in particular however (mono) or (di) sodium, (mono) or (di)potassium, magnesium or calcium salts.

The term salt formed with a physiologically compatible cation is understood within the meaning of the present invention to denote salts of at least one of the respective compounds as anion with at least one inorganic cation, which are physiologically compatible, especially when used in humans and/or mammals. Particularly preferred are the salts of the alkali and alkaline earth metals, but also NH₄⁺, in particular however (mono) or (di)sodium, (mono) or (di)potassium, magnesium or calcium salts.

The compounds used according to the invention and their preparation are in principle known from DE 44 26 245 A1 with regard to the 1-phenyl-3-dimethylaminopropane compounds according to the general Formula I. All compounds other than these specific compounds can easily be prepared by the person skilled in the art in a similar way to the synthesis pathways described there.

In a particularly preferred variant of this embodiment, with regard to the 1-phenyl-3-dimethylaminopropane compounds of the general Formula I used according to the invention where R³=H, these are present in the form of the diastereomers with the relative configuration Ia 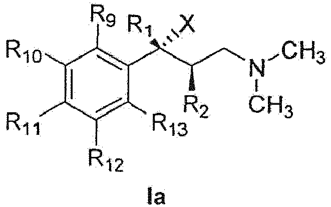 in particular in mixtures with a larger proportion of this diastereomer compared to the other diastereomer, or are used as pure diastereomer.

It is particularly preferred if the 1-phenyl-3-dimethylaminopropane compound of the general Formula I used according to the invention is selected from the following group:
- (2RS,3RS)-1-dimethylamino-3-(3-methoxyphenyl)-2-methyl-pentan-3-ol,
- (+)-(2R, 3R)-1-dimethylamino-3-(3-methoxyphenyl)-2-methyl-pentan-3-ol, and
- (2R,3R)-1-dimethylamino-3-(3-methoxyphenyl)-2-methyl-pentan-3-ol,
- (-)-(2S,3S)-1-dimethylamino-3-(3-methoxyphenyl)-2-methyl-pentan-3-ol,
- (2S,3S)-1-dimethylamino-3-(3-methoxyphenyl)-2-methyl-pentan-3-ol,
- (2RS,3RS)-3-(3,4-dichlorophenyl)-1-dimethylamino-2-methyl-pentan-3-ol,
- (2RS,3RS)-3-(3-difluoromethylphenyl)-1-dimethylamino-2-methyl-pentan-3-ol,
- (2RS, 3RS)-1-dimethylamino-2-methyl-3-(3-methylsulfanylphenyl)-pentan-3-ol,
- (3RS)-1-dimethylamino-3-(3-methoxyphenyl)-4,4-dimethylpentan-3-ol,
- (2RS,3RS)-3-(3-dimethylamino-1-ethyl-1-hydroxy-2-methylpropyl)-phenol,
- (1RS, 2RS)-3-(3-dimethylamino-1-hydroxy-1,2-dimethylpropyl)-phenol,
- (+)-(1R, 2R)-3-(3-dimethylamino-1-hydroxy-1,2-dimethylpropyl)-phenol,
- (1R, 2R)-3-(3-dimethylamino-1-hydroxy-1,2-dimethylpropyl)phenol,
- (-)-(1S, 2S)-3-(3-dimethylamino-1-hydroxy-1,2-dimethylpropyl)-phenol,
- (1S, 2S)-3-(3-dimethylamino-1-hydroxy-1,2-dimethylpropyl)phenol,
- (RS, RS)-3-(3-dimethylamino-1-ethyl-2-methylpropyl)-phenol,
- (-)-(1R, 2R)-3-(3-dimethylamino-1-ethyl-2-methylpropyl)phenol,
- (1R, 2R)-3-(3-dimethylamino-1-ethyl-2-methylpropyl)-phenol,
- (+)-(1S, 2S)-3-(3-dimethylamino-1-ethyl-2-methylpropyl)phenol,
- (1S,2S)-3-(3-dimethylamino-1-ethyl-2-methylpropyl)-phenol,
- (+)-(1R, 2R)-acetic acid-3-dimethylamino-1-ethyl-1-(3-methoxy-phenyl)-2-methyl propyl ester,
-
- (2RS, 3R5)-3-(4-chlorophenyl)-1-dimethylamino-2-methylpentan-3-ol,
- (+)-(2R, 3R)-3-(3-dimethylamino-1-ethyl-1-hydroxy-2-methylpropyl)-phenol,
- (2RS, 3RS)-4-dimethylamino-2-(3-methoxyphenyl)-3-methylbutan-2-ol and
- (+)-(2R, 3R)-4-dimethylamino-2-(3-methoxyphenyl)-3-methylbutan-2-ol,
   preferably as the hydrochloride,
   in particular
- (RS, RS)-3-(3-dimethylamino-1-ethyl-2-methylpropyl)-phenol,
- (-)-(1R,2R)-3-(3-dimethylamino-1-ethyl-2-methylpropyl)phenol,
- (1R, 2R)-3-(3-dimethylamino-1-ethyl-2-methylpropyl) phenol,
- (-)-(1S, 2S)-3-(3-dimethylamino-1-ethyl-2-methylpropyl)phenol,
- (1S, 2S)-3-(3-dimethylamino-1-ethyl-2-methylpropyl) phenol,
   preferably
- (-)-(1R,2R)-3-(3-dimethylamino-1-ethyl-2-methylpropyl)phenol,
- (1R,2R)-3-(3-Dimethylamino-1-ethyl-2-methylpropyl)-phenol.

The medicaments for treating rheumatoid, preferably rheumatoid arthritic, very preferably chronic rheumatoid arthritic pain for the preparation of which the aforementioned compounds are used according to the invention, contain at least one aforementioned active constituent used according to the invention, as well as optionally suitable additives and/or auxiliary substances.

Suitable additives and/or auxiliary substances within the meaning of the present invention are all substances known to the person skilled in the art from the prior art for producing galenical formulations. The choice of these auxiliary substances as well as the amounts thereof to be used depend on whether the medicament is to be applied orally, intravenously, intraperitonealy, intradermally, intramuscularly, intranasally, buccally or topically. For oral application suitable preparations are in the form of tablets, chewable tablets, coated pills, capsules, granules, drops, juices or syrups, while for parenteral, topical and inhalative application suitable preparations are solutions, suspensions, readily reconstitutable dry preparations as well as sprays. A further possibility are suppositories for rectal use. The use in a depot in dissolved form, in a carrier film or a plaster, optionally with the addition of agents promoting penetration of the skin, are examples of suitable percutaneous application forms. Examples of auxiliary substances and additives for oral application forms are disintegrants, lubricants, binders, fillers, mould release agents, optionally solvents, taste enhancers, sugars, in particular carriers, diluents, colourants, antioxidants, etc. For suppositories there may be used inter alia waxes or fatty acid esters, and for parenteral application agents there may be used carriers, preservatives, suspension aids, etc. The amounts of active constituent to be administered to patients vary depending on the patient's weight, type of application, and the severity of the medical condition. The compounds according to the invention may be released in a delayed manner from orally, rectally or percutaneously usable preparation forms. In the medical indications for use according to the invention corresponding retard formulations, in particular in the form of a "once daily" preparation, which need to be taken only once a day, are especially preferred.

Preferred are medicaments that contain at least 0.05 to 90.0% of the active constituent, in particular low active dosages, in order to avoid side effects. Normally 0.1 to 5000 mg/kg, in particular 1 to 500 mg/kg and preferably 2 to 250 mg/kg of body weight of at least one compound used according to the invention are administered. However, the administration of 0.01 - 5 mg/kg, preferably 0.03 to 2 mg/kg and especially 0.05 to 1 mg/kg of body weight is also preferred and customary.

Examples of auxiliary substances include the following: water, ethanol, 2-propanol, glycerol, ethylene glycol, propylene glycol, polyethylene glycol, polypropylene glycol, glucose, fructose, lactose, sucrose, dextrose, molasses, starch, modified starch, gelatin, sorbitol, inositol, mannitol, microcrystalline cellulose, methylcellulose, carboxymethylcellulose, cellulose acetate, shellac, cetyl alcohol, polyvinylpyrrolidone, paraffins, waxes, natural and synthetic gums, acacia gum, alginates, dextran, saturated and unsaturated fatty acids, stearic acid, magnesium stearate, zinc stearate, glyceryl stearate, sodium lauryl sulfate, edible oils, sesame oil, coconut oil, ground nut oil, soya bean oil, lecithin, sodium lactate, polyoxyethylene and polyoxypropylene fatty acid esters, sorbitan fatty acid esters, sorbic acid, benzoic acid, citric acid, ascorbic acid, tannic acid, sodium chloride, potassium chloride, magnesium chloride, calcium chloride, magnesium oxide, zinc oxide, silicon dioxide, titanium oxide, titanium dioxide, magnesium sulphate, zinc sulphate, calcium sulphate, potassium carbonate, calcium phosphate, dicalcium phosphate, potassium bromide, potassium iodide, talcum, kaolin, pectin, crospovidone, agar and bentonite.

The preparation of these medicaments and pharmaceutical compositions is carried out with the aid of agents, equipment, methods and processes well known in the prior art for pharmaceutical formulations, such as are described for example in "Remington's Pharmaceutical Sciences", edited by A.R. Gennaro, 17th Ed., Mack Publishing Company, Easton, Pa (1985), in particular in Part 8, Chapters 76 to 93.

Thus, for example, for a solid formulation such as a tablet, the active constituent of the medicament can be granulated with a pharmaceutical carrier, for example conventional tablet constituents such as maize starch, lactose, sucrose, sorbitol, talcum, magnesium stearate, dicalcium phosphate or pharmaceutically acceptable gums, and pharmaceutical diluents, such as for example water, in order to form a solid composition that contains the active constituent in homogeneous distribution. A homogeneous distribution is understood here to mean that the active constituent is distributed uniformly over the whole composition, so that the latter can be subdivided without any problem into identically active unit dose forms such as tablets, pills or capsules. The solid composition is then subdivided into unit dose forms. The tablets or pills of the medicament according to the invention or of the compositions according to the invention can also be coated or compounded in some other way so as to produce a dose form having delayed release. Suitable coating agents are inter alia polymeric acids and mixtures of polymeric acids with materials such as for example schellac, cetyl alcohol and/or cellulose acetate.

Even if the medicaments prepared according to the invention exhibit only slight side effects, it can for example be advantageous, in order to avoid certain forms of dependence, to employ apart from the aforementioned compound according to the invention also morphine antagonists, in particular naloxone, naltrexone and/or levallorphan.

The invention also relates to a method for treating rheumatoid, preferably rheumatoid arthritic, very preferably chronic rheumatoid arthritic pain, in which at least one of the aforementioned compounds is used according to the invention.

The following examples are intended to describe the invention in more detail, without however restricting the subject-matter of the invention.

### Example

The compound Tapentadol ((-)-(1R,2R)-3-(3-dimethylamino-1-ethyl-2-methyl-propyl)¬phenol) was tested and is hereinafter abbreviated as compound (or Comp.) 1

A preclinical model for rheumatoid pain is used according Wilson et al., Pain 2006

The experiments were carried out in male albino rats (Sprague Dawley) with 135 - 170 g body weight. All rats were used only once. Rheumatoid arthritis was induced by intra-articular injection of CFA in one knee joint of a rat hindpaw. For this purpose the rats were anaesthetised using 3% isoflurane in oxygen. The left knee was cleaned using a Cutasept□F solution. The left knee of each rat was injected with 150 µl of CFA; containing 2 mg/ml Mycobacterium tuberculosis. The right joints were untreated. Animals were assessed for changes in weight bearing five days post intraarticular injection.

Naive rats distribute their body weight equally between their two hind legs. After induction of arthric inflammatory pain, the weight is redistributed such that less weight is placed on the affected leg. Weight bearing on each hind leg was determined using a rat incapacitance tester (Somedic Sales AB, Hörby, Sweden). Rats were placed in an angled plexiglas chamber of the incapacitance tester with their hind paws on separate sensors, and the percentage body weight distribution was calculated over a period of 30 s. Data were expressed as percentage of contralateral weight bearing, with 100% values resulting from equal weight distribution across both hind limbs.

The present study was designed to investigate the analgesic effects of Tapentadol in chronic knee joint arthritic pain in rats after intravenous (i.v.) application. Oxycodone was tested as comparator.

### Results:

Tapentadol significantly reduced the CFA-induced decrease in weight bearing in a dose dependent manner, with a maximal effect of 51.0 ± 11.2 % at the dose of 4.64 mg/kg (i.v.). The analgesic efficacy of Tapentadol was close to the comparators morphine (59.6 % at the dose of 2.15 mg/kg) ibuprofen (54.7 % efficacy at the dose of 147 mg/kg) and oxycodone (46,1 % efficacy at the dose of 0,464 mg/kg) Higher doses of the tested compounds Tapentadol, morphine, ibuprofen and oxycodone resulted in readout (weight bearing) confounding side effects and were not analyzed.

Analgesic effect of Tapentadol, morphine, ibuprofen and oxycodone on CFA-induced chronic arthritic pain. Data are expressed as mean percentage of maximal possible effect ± S.E.M at the highest possible dose without readout confounding side effects. (n = 10):

| **Compound** | **Analgesic efficacy @ dose [mg/kg]** |
|---|---|
| Tapentadol (iv) | 51.0 % @ 4.64 mg/kg |
| Morphine (iv) | 59.6 % @ 2.15 mg/kg |
| Ibuprofen (ip) | 54.7 % @ 147 mg/kg |
| Oxycodone (i.v.) | 46,1 % @ 0,464 mg/kg |

| | |
|---|---|
| iv = intravenous application; ip = intraperitoneal application | |

### Literature:

Colpaert FC. Evidence that adjuvant arthritis in the rat is associated with chronic pain. Pain. 1987; 28(2):201-22.

### Review

Pearson CM, Wood FD. Studies of arthritis and other lesions induced in rats by the injection of mycobacterial adjuvant. VII. Pathologic details of the arthritis and spondylitis. Am J Pathol 1963; 42:73-95

Hu SJ, Zhu J. Sympathetic facilitation of sustained discharges of polymodal nociceptors. Pain. 1989; 38(1):85-90

Schaible HG, Schmidt RF. Time course of mechanosensitivity changes in articular afferents during a developing experimental arthritis. J Neurophysiol. 1988; 60(6):2180-2195

Wilson AW, Medhurst SJ, Dixon CI, Bontoft NC, Winyard LA, Brackenborough KT, De Alba J, Clarke CJ, Gunthorpe MJ, Hicks GA, Bountra C, McQueen DS, Chessell IP. An animal model of chronic inflammatory pain: pharmacological and temporal differentiation from acute models. Eur J Pain. 2006; 10(6):537-549

## Claims

1. Use of a 1-phenyl-3-dimethylamino-propane compound according to the general Formula I 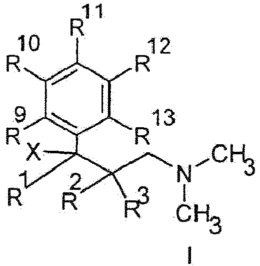
wherein X is selected from OH, F, Cl, OC(O)CH₃ or H, preferably OH, F, OC(O)CH₃ or H,
and/or
R¹ is selected from C₁₋₄-alkyl, saturated and unsubstituted, branched or unbranched; preferably CH₃, C₂H₅, C₄H₉ or t-butyl, in particular CH₃ or C₂H₅,
and/or
R² and R³ independently of one another are selected from H, C₁₋₄-alkyl, saturated and unsubstituted, branched or unbranched; preferably H, CH₃, C₂H₅, i-propyl or t-butyl, in particular H or CH₃, preferably R³ = H,
or
R² and R³ together form a C₅₋₆-cycloalkyl radical, saturated or unsaturated, unsubstituted or monosubstituted or polysubstituted, preferably saturated and unsubstituted, in particular cyclohexyl, and/or
R⁹ to R¹³, in which three or four of the radicals R⁹ to R¹³ must correspond to H, are independently of one another selected from H, Cl, F, OH, CF₂H, CF₃ or C₁₋₄-alkyl, saturated and unsubstituted, branched or unbranched; OR¹⁴ or SR¹⁴, where R¹⁴ is selected from C₁₋₃-alkyl, saturated and unsubstituted, branched or unbranched;
preferably H, Cl, F, OH, CF₂H, CF₃, OCH₃ or SCH₃
or R¹² and R¹¹ form a 3,4-OCH=CH ring,
in particular
if R⁹, R¹¹ and R¹³ correspond to H, one of R¹⁰ and R¹² also corresponds to H, while the other is selected from:
Cl, F, OH, CF₂H, CF₃, OR¹⁴ or SR¹⁴, preferably OH, CF₂H, OCH₃ or SCH₃,
or
if R⁹ and R¹³ correspond to H and R¹¹ corresponds to OH, OCH₃, Cl or F, preferably to Cl, then one of R¹⁰ and R¹² also corresponds to H, while the other corresponds to OH, OCH₃, Cl or F, preferably Cl,
or
if R⁹, R¹⁰, R12 and R¹³ correspond to H, R¹¹ is selected from CF₃, CF₂H, Cl or F, preferably F,
or
if R¹⁰, R¹¹ and R¹² correspond to H, one of R⁹ and R¹³ also corresponds to H, while the other is selected from OH, OC₂H₅ or OC₃H₇,
optionally in the form of their racemates, their pure stereoisomers, in particular enantiomers or diastereomers, or in the form of mixtures of the stereoisomers, in particular of the enantiomers or diastereomers, in an arbitrary mixture ratio; in the prepared form or in the form of their acids or their bases or in the form of their salts, in particular the physiologically compatible salts, or in the form of their solvates, in particular the hydrates;
for the production of a medicament for treating rheumatoid, preferably rheumatoid arthritic, very preferably chronic rheumatoid arthritic pain.

2. Use according to claim 1, **characterised in that** the compound of the Formula I where R³ = H is present in the form of the diastereomers with the relative configuration Ia 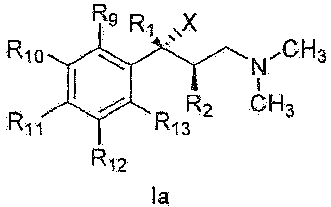 is used in particular in mixtures with a higher proportion of this diastereomer compared to the other diastereomer, or is used as pure diastereomer.

3. Use according to one of claims 1 and 2, **characterised in that** a compound of the Formula I selected from the following group is used:
• (2RS,3RS)-1-dimethylamino-3-(3-methoxyphenyl)-2-methyl-pentan-3-ol,
• (+)-(2R, 3R)-1-dimethylamino-3-(3-methoxyphenyl)-2-methyl-pentan-3-ol, and
• (2R,3R)-1-dimethylamino-3-(3-methoxyphenyl)-2-methylpentan-3-ol,
• (-)-(2S,3S)-1-dimethylamino-3-(3-methoxyphenyl)-2-methyl-pentan-3-ol,
• (2S,3S)-1-dimethylamino-3-(3-methoxyphenyl)-2-methylpentan-3-ol,
• (2RS,3RS)-3-(3,4-dichlorophenyl)-1-dimethylamino-2methyl-pentan-3-ol,
• (2RS,3RS)-3-(3-difluoromethylphenyl)-1-dimethylamino-2methyl-pentan-3-ol,
• (2RS,3RS)-1-dimethylamino-2-methyl-3-(3-methylsulfanylphenyl)-pentan-3-ol,
• (3RS)-1-dimethylamino-3-(3-methoxyphenyl)-4,4-dimethylpentan-3-ol,
• (2RS,3RS)-3-(3-dimethylamino-1-ethyl-1-hydroxy-2-methylpropyl)-phenol,
• (1RS, ZRS)-3-(3-dimethylamino-1-hydroxy-1,2-dimethylpropyl)-phenol,
• (+)-(1R, 2R)-3-{3-dimethylamino-1-hydroxy-1,2-dimethylpropyl)-phenol,
• (1R, 2R)-3-(3-dimethylamino-1-hydroxy-1,2-dimethylpropyl)phenol,
• (-)-(1S, 2S)-3-(3-dimethylamino-1-hydroxy-1,2-dimethylpropyl)-phenol,
• (1S, 2S)-3-(3-dimethylamino-1-hydroxy-1,2-dimethylpropyl)phenol,
• (RS, RS)-3-(3-dimethylamino-1-ethyl-2-methylpropyl)phenol,
• (-)-(1R, 2R)-3-(3-dimethylamino-1-ethyl-2-methylpropyl)phenol,
• (1R, 2R)-3-(3-dimethylamino-1-ethyl-2-methylpropyl)phenol,
• (+)-(1S, 2S)-3-(3-dimethylamino-1-ethyl-2-methylpropyl)phenol,
• (1S,2S)-3-(3-dimethylamino-1-ethyl-2-methylpropyl)phenol,
• (+)-(1R, 2R)-acetic acid-3-dimethylamino-1-ethyl-1-(3-methoxy-phenyl)-2-methyl propyl ester,
• (2RS, 3RS)-3-(4-chlorophenyl)-1-dimethylamino-2-methylpentan-3-ol,
• (+)-(2R, 3R)-3-(3-dimethylamino-1-ethyl-1-hydroxy-2-methylpropyl)-phenol,
• (2RS, 3RS)-4-dimethylamino-2-(3-methoxyphenyl)-3methylbutan-2-ol and
• (+)-(2R, 3R)-4-dimethylamino-2-(3-methoxyphenyl)-3-methylbutan-2-ol,
preferably as the hydrochloride,
in particular
• (RS, RS)-3-(3-dimethylamino-1-ethyl-2-methylpropyl)phenol,
• (-)-(1R,2R)-3-(3-dimethylamino-1-ethyl-2-methylpropyl) phenol,
• (1R, 2R)-3-(3-dimethylamino-1-ethyl-2-methylpropyl) phenol,
• (-)-(1S, 2S)-3-(3-dimethylamino-1-ethyl-2-methylpropyl) phenol,
• (1S, 2S)-3-(3-dimethylamino-1-ethyl-2-methylpropyl) phenol,
preferably
• (-)-(1R,2R)-3-(3-dimethylamino-1-ethyl-2-methyl-propyl)phenol,
• (1R,2R)-3-(3-Dimethylamino-1-ethyl-2-methyl-propyl) phenol.

4. Use of (1RS, 2RS)-3-(3-dimethylamino-1-ethyl-2-methylpropyl)-phenol, preferably (1R, 2R)-3-(3-dimethylamino-1-ethyl-2-methylpropyl)-phenol, most preferably as the hydrochloride salt, for the preparation of a medicament for treating rheumatoid, preferably rheumatoid arthritic, very preferably chronic rheumatoid arthritic pain.

5. Use of compounds of the Formula I according to claims 1-3, preferably of the Formula Ia, most preferably the compound (1RS, 2RS)-3-(3-dimethylamino-1-ethyl-2-methylpropyl)-phenol, most particularly preferably (1R, 2R)-3-(3-dimethylamino-1-ethyl-2-methylpropyl)-phenol, for treating rheumatoid, preferably rheumatoid arthritic, very preferably chronic rheumatoid arthritic pain.

6. Use of compounds according to claims 1-3, preferably (1RS, 2RS)-3-(3-dimethylamino-1-ethyl-2-methylpropyl)-phenol, most preferably (1R, 2R)-3-(3-dimethylamino-1-ethyl-2-methylpropyl)-phenol, for treating rheumatoid, preferably rheumatoid arthritic, very preferably chronic rheumatoid arthritic pain.

7. Compounds according claim 1-3 preferably (1RS, 2RS)-3-(3-dimethylamino-1-ethyl-2-methylpropyl)-phenol, most preferably (1R, 2R)-3-(3-dimethylamino-1-ethyl-2-methylpropyl)-phenol for treating rheumatoid, preferably rheumatoid arthritic, very preferably chronic rheumatoid arthritic pain.
